(19) Europäisches Patentamt
European Patent Office
Office européen des brevets

(11) **EP 1 704 506 B1**

(12) **EUROPEAN PATENT SPECIFICATION**

(45) Date of publication and mention
of the grant of the patent:
**22.04.2009 Bulletin 2009/17**

(51) Int Cl.:
***G06F 19/00*** (2006.01)

(21) Application number: **05701874.9**

(22) Date of filing: **14.01.2005**

(86) International application number:
**PCT/GB2005/000106**

(87) International publication number:
**WO 2005/068089 (28.07.2005 Gazette 2005/30)**

(54) **MULTIPLE INEXACT PATTERN MATCHING**

MEHRFACHER UNGENAUER MUSTERVERGLEICH

CONCORDANCE INEXACTE MULTIPLE

(84) Designated Contracting States:
**AT BE BG CH CY CZ DE DK EE ES FI FR GB GR
HU IE IS IT LI LT LU MC NL PL PT RO SE SI SK TR**

(30) Priority: **16.01.2004 GB 0400974**

(43) Date of publication of application:
**27.09.2006 Bulletin 2006/39**

(73) Proprietor: **Illumina Cambridge Limited
Little Chesterford
Saffron Walden
Essex CB10 1XL (GB)**

(72) Inventor: **COX, Anthony, James,
Illumina Cambridge Limited
Saffron Walden,
Essex CB10 1XL (GB)**

(74) Representative: **Palmer, Jonathan R.
Boult Wade Tennant,
Verulam Gardens,
70 Gray's Inn Road
London WC1X 8BT (GB)**

(56) References cited:
**WO-A-01/48637**          **US-A- 5 577 249**

• MUTH R ET AL: "Approximate multiple string
search" COMBINATORIAL PATTERN
MATCHING. 7TH ANNUAL SYMPOSIUM, CPM 96.
PROCEEDINGS SPRINGER-VERLAG BERLIN,
GERMANY, 1996, pages 75-86, XP002359701
ISBN: 3-540-61258-0 cited in the application
• JIONG YANG ET AL: "Accelerating approximate
subsequence search on large protein sequence
databases" PROCEEDINGS IEEE COMPUTER
SOCIETY BIOINFORMATICS CONFERENCE IEEE
COMPUT. SOC LOS ALAMITOS, CA, USA, 2002,
pages 1-10, XP002359702 ISBN: 0-7695-1653-X
• BENG CHIN OOI ET AL: "Fast filter-and-refine
algorithms for subsequence selection"
PROCEEDINGS INTERNATIONAL DATABASE
ENGINEERING AND APPLICATIONS
SYMPOSIUM IEEE COMPUT. SOC PISCATAWAY,
NJ, USA, 2002, pages 243-254, XP002359703
ISBN: 1098-8068

## Description

## Introduction

The present invention relates to methods and apparatus for seeking, in a target sequence, an exact or inexact match to a given query sequence. In particular, but not exclusively, the invention relates to such methods and apparatus for use in searching for exact or close matches to each of a large number of genetic data query sequences in a large amount of genetic sequence target data such as a whole genome.

## Discussion of the prior art

Computer implemented methods to efficiently search for one or more query data sequences in a target sequence dataset are used in a variety of fields, including searching for string query sequences in large numbers of general or text computer data files using tools such as UNIX grep and agrep, Internet Web searches using search engines such as Google (RTM) and searches for short sequences of polynucleotide or polypeptide data in large genome databases.

Wu and Manber, "A Fast Algorithm for Multi-Pattern Searching", Tech Report TR-94-17, Department of Computer Science, University of Arizona, 1994, discloses a computer implemented method which simultaneously searches for exact matches between multiple query sequences and a target data set much more efficiently than can be achieved by carrying out corresponding multiple separate searches each for a single query sequence. Examples apply the method to searching large text files for hundreds of different English language words simultaneously.

In the method of Wu and Manber, a hash table is built, indexed by the hash value of all possible values of a particular end segment of the query sequences. Each entry in the hash table contains or points to query sequences ending in the corresponding segment. Generally, many entries in the table will be empty, while some entries will point to multiple query sequences. The target sequence is scanned. For each possible matching segment of the target sequence a hash value is generated and used to look up potential query sequence matches using the hash table. Various other techniques, such as the use of a shift table, are used to improve efficiency.

Kim and Kim, "A fast multiple string-pattern matching algorithm", Proc. of 17th AoM/IaoM Conf. on Computer Science, Aug 1999, discloses a broadly similar computer implemented method, but applied to searching for exact matches in a DNA target data set, as well as an English language text target data set. Each nucleotide base type, A, T, G and C, is ascribed a different two bit code, 00, 01, 10 and 11. Query sequences of nucleotide bases (eg TGCAGG) are thereby converted to binary query sequences (eg 011011001010). Each binary query sequence is split into a suffix and a prefix (eg 011011 and 001010). A hash function is applied to each prefix, and the hash value is used as an index into a hash table containing the corresponding suffixes.

A target sequence of nucleotide bases is scanned, base by base. At each position a target fragment of the same size as each query sequence is extracted, binary encoded and divided into prefix and suffix. The hash function is applied to the prefix and the result used as an index into the hash table to obtain the related query sequence suffixes, each of which is compared with the target fragment suffix to seek an exact match. The suffix comparison is straightforward and reasonably fast because the test is for an identical match.

Muth and Manber, "Approximate Multiple String Search", Proc. of 7th Annual Symposium on Combinatorial Pattern Matching", pp75-86, 1996, discloses a computer implemented method for rapidly searching a large target text database for multiple query text strings, allowing for an error of one different character in finding an inexact match. Errors of one different character may be by deletion, insertion or substitution of a character. The algorithm uses a hash table approach similar to that discussed above. However, each query sequence is copied several times and each copy is modified by omitting a different character. The target database is scanned, and each possible matching target segment is processed in the same way as each query sequence. All the modified target segment copies are compared against all the modified query sequences.

The Muth and Manber method for inexact matching is relatively inefficient even when searching for matches subject to just one error, although better than the simplest multiple inexact matching method of generating all possible error perturbations of each query which should be considered a match, and feeding these to an exact matching mechanism. For a five character query sequence, five modified query copies must be compared, using the Muth and Manber method, against five modified copies of each possible target segment. When two errors are to be considered the number of different combinations which must be considered makes the technique impractical.

It would be desirable to provide an improved computer implemented method and associated apparatus for seeking matches between a large number of query sequences and a large target data set, allowing for a restricted number of differences, or mismatches between a query sequence and a matching target fragment.

It would particularly be desirable to provide such a method and apparatus which reduces the processing time required to make the necessary comparisons.

## Summary of the Invention

The invention seeks to address the above and other problems of the related prior art. Generally, computer implemented methods of seeking matches between one or more query sequences and one or more target sequences, allowing for limited differences between the query and target sequences, are provided. Also provided are computer apparatus appropriately arranged or programmed to carry out such methods and computer readable media carrying corresponding program code. In particular, the invention provides a computer implemented method of searching for a plurality of query sequences in a set of target sequence fragments as set out in claim 1, and an apparatus as set out in claim 17,

The target sequences will usually be overlapping fragments of one or more longer target sequences, each target fragment selected to be the same size as each query sequence.

The invention is particularly useful when applied to the problem of seeking inexact matches between large numbers of query sequences and the target data. In the described embodiments, the invention is used in seeking inexact matches between a large number of short nucleic acid sequences, typically each between about 16 and 32 bases in length, and genome data, allowing for up to two sequence differences between each query and a matching genome fragment. Such an application is important in analysing the large amounts of nucleic acid data produced very rapidly using contemporary and envisaged genome analysis techniques.

The invention is implemented by dividing each query sequence into segments, distributing these segments between first and second segment groups, and using an efficient algorithm to seek potential matches between a first group of segments of each query sequence and a corresponding first group of segments of each target sequence fragment. This step is repeated using different distributions of segments from each query sequence and target sequence fragment in the compared first groups. The different distributions are arranged such that, whatever the positions of the potential mismatches between a target sequence fragment and a query sequence being compared, at least one distribution will place all mismatches, up to a certain number, into the second groups. Having found potential matches using a rapid comparison of the first groups, a detailed comparison of the corresponding second groups establishes the required exact or inexact matches.

The invention may be used with query sequence divided into just two segments, to seek matches subject to just one sequence difference, but is preferably used with four segments evenly distributed between the first and second groups to find matches subject to up to two sequence differences. In specific embodiments, the first and second groups are referred to as prefix and suffix, but the implied ordering is clearly not essential.

In particular, embodiments provide a method of searching for a plurality of query sequences in a set of target sequence fragments, allowing for mismatches at up to n sequence positions, comprising the steps of:

a) logically dividing each query sequence and each target fragment into at least n+1 segments;
b) for each query sequence, constructing a first and a second query group by distributing the query sequence segments there between such that at least n segments are contained in the second query group. Embodiments also provide:
c) constructing from each target fragment a first target group having the same segment distribution as the first query group; and
d) for each query sequence, comparing said first query group with each first target group to identify potential matching target fragments. The method may then proceed to find actual matching target fragments, subject to the mismatches to be allowed for, by comparing the second groups or by comparing directly the potentially matching query sequence and target fragment, parts or derivations thereof.

The segments may be formed from simple splitting of the elements of each sequence, or a more complex derivation of segments could be used involving for example, coding or scrambling. In particular, a segment does not need to be formed from sequence items which are consecutive in the original sequence. For example, a sequence 123456 could be segmented as 123:456 or 135:246 or 621:435 and so on.

Preferably, all segments are the same size as each other, or as nearly the same size as possible given the number of segments and the size of the sequence being split.

Likewise, first and second query and target groups of segments are most conveniently formed using a simple concatenation of segments, in an arbitrary order, but more complicated schemes involving coding, convolution, scrambling and so on could be used. The relative sizes of the first and second groups may be optimized having regard to the available computer memory and other resources and features of the data. In the embodiments described, equal sizes of first and second groups is preferable, and allows for certain additional optimizations.

Each different or distinct distribution of segments into the first and second groups may be described as a hash function. To distribute four segments into two groups there are six such distinct hash functions, bearing in mind that the order in which the segments are placed in a group is immaterial, as long as the ordering is consistent for the purposes of comparing groups. Preferably, said query sequences and target sequence fragments are logically divided into an even number of segments, and the segments are distributed in equal numbers between the first and second query and target groups of segments.

To find all potential matches, steps (b) to (d) are repeated using different distributions of segments between said query groups, ideally using all distinct distributions of segments between said query groups, although it may not be necessary to use all distinct combinations to find at least one match.

For each potential matching target fragment identified in step (d), the following steps are carried out:

(i) constructing a second target group having the same segment distribution as the second query group of the potentially matching query sequence; and

(ii) comparing said second query group with said second target group to identify a match, allowing for mismatches in up to n sequence positions.

Step (ii) may be efficiently carried out by applying an exclusive OR (XOR) operation between the second query group and second target group. This is applicable, in particular, if the sequences are encoded as binary numbers, for example using the 2-bit encoding to represent each of the four possible nucleotides in a DNA sequence. By first constructing a lookup table associating each possible outcome of the XOR operation with a match output, which may include a boolean match flag, a number of matches and so on, the output of the XOR operation may be processed more rapidly.

The method may be carried out by constructing a first query table indexed by possible values of the first query groups, the entries in the first query table providing access to each second query group by using as an index the value of a corresponding first query group. This first query table may be described as a hash table, as it represents a mapping between potential first query groups and actual second query groups according to one of the distinct segment distributions, or hash functions.

For each distinct distribution of query sequence segments, or hash function, there may then be constructed a second query table providing access to each second query group, the entries in the first query table providing references to appropriate entries in the second query table. For each first target group constructed in step (c), step (d) is preferably implemented by using said first target group to form an index into said first query table. Of course, the tables may be constructed in a variety of ways using any appropriate data structures such as lists, arrays and so on, which may be compressed or compacted as desired. The indexing and referencing may be achieved using pointers, direct numerical indexes, second level hashing and a variety of other techniques familiar to the skilled person.

If two distinct distributions of query sequence segments (or hash functions) are such that the first query group of one distribution is the same as the second query group of the other distribution and vice versa, the query tables for both of the distinct distributions may be constructed and/or used concurrently, simply by swapping first and second groups of data at appropriate points. This technique may be used to reduce the number of passes required through the target data by a factor of two.

The embodiments also provide computer apparatus, comprising a memory, for searching for a plurality of query sequences in a set of target sequence fragments, allowing for mismatches at up to n sequence positions comprising: a query groups constructor adapted to construct in the memory, for each query sequence, first and second query groups by logically dividing each query sequence into **n+1** segments and distributing the query sequence segments between the first and second query groups in one or more ways such that at least n segments are contained in each second query group; a target groups constructor adapted to construct in the memory, for each target sequence fragment, one or more first target groups having segment distributions corresponding to the first query groups; and a first group comparator adapted, for each query sequence, to compare said one or more first query groups with corresponding ones of said one or more first target groups and to thereby output a result identifying potentially matching target fragments.

Such apparatus may typically be provided by a personal or desk top computer having a visual display unit, input devices, a central processing unit, volatile and non volatile memory and so on. The software for carrying out the method may be provided on removable media such as a CD ROM, or loaded over a network connection. Query and target data may be loaded and/or stored locally, and/or obtained over a network connection.

**Brief Description of the Drawings**

Embodiments of the invention will now be described, by way of example only, with reference to the accompanying drawings, of which:

Figure 1 illustrates steps of a method embodying the invention;
Figure 2 illustrates in more detail the build hash tables step of figure 1;
Figure 3 illustrates the mask repeats step of figure 2;
Figure 4 illustrates the count prefixes step of figure 2;
Figure 5 illustrates the hash query sequences step of figure 2;
Figure 6 illustrates the scan chromosome step of figure 1;
Figure 7 illustrates the search for prefix, suffix step of figure 6;
Figure 8 illustrates the search for suffix, prefix step of figure 6;
Figure 9 illustrates apparatus embodying the invention; and
Figure 10 illustrates data structures formed in memory by the apparatus of figure 9.

**Detailed description of preferred embodiments**

A general explanatory embodiment of the invention will first be provided, omitting complex implementation details. The embodiment provides a computer implemented method for seeking matches of a plurality of query sequences of nucleotide bases in a target sequence of nucleotide bases, or indeed a set or database of such sequences, allowing matching with up to 2 substitution errors, whereby a query sequence and a fragment of a target sequence differ at up to two positions.

Each query sequence is partitioned into four segments A, B, C and D, each containing a roughly equal number of bases. A function is defined $f_i(ABCD) \rightarrow (P, S)$ that forms a first group, which will be referred to as a prefix, from two of these four segments and a second group, or suffix, from the remaining two. If a query sequence has been binary encoded, it is straightforward to implement such a function using bit masking and bit shifting operations. Neglecting the ordering of bases within prefixes and suffixes, there are six such distinct functions:

$$f_1(ABCD) \rightarrow (AB,CD)$$

$$f_2(ABCD) \rightarrow (CD,AB)$$

$$f_3(ABCD) \rightarrow (AC,BD)$$

$$f_4(ABCD) \rightarrow (BD,AC)$$

$$f_5(ABCD) \rightarrow (AD,BC)$$

$$f_6(ABCD) \rightarrow (BC,AD)$$

Six passes are made through the target sequence. During each pass a different one of the above hash functions $f_1 ... f_6$ is used. At each pass a hash table process similar to that described above in respect of the Kim and Kim document is used, except that in the present embodiment the hash function is used to split query sequences and target sequence fragments into prefixes and suffixes.

The first step of pass $i$ is to place the suffix of each query sequence, computed by $f_i$, in a hash table using the corresponding prefix as key. The target database is then scanned, base by base. At each base a target fragment is extracted and $f_i$ is used to generate the target fragment prefix and suffix. The prefix of the target fragment is used as a key into the hash table, to obtain a set of query sequence suffixes that are to be compared with the suffix of the target fragment.

If a target fragment is an exact match to a query sequence, then all six the hash functions $f_{1..6}$ will generate identical prefixes for both the target fragment and the query sequence. Consider, instead, an inexact match between a target fragment and a query sequence, with differences at two base positions. If both differences occur in the same segment, then identical prefixes will be generated for the target fragment and query sequence by three of the six hash functions. The only other possibility is that the differences occur in two different segments, in which case one of the six hash functions will still generate identical prefixes for the target fragment and query sequence. Thus, whatever the position of the two differences between the target sequence fragment and the query sequence, at least one of the six hash functions yields an identical prefix for both sequences. For example, if the two differences lie in segments A and C then both differences lie in the suffix of $f_4$.

On finding an exact match between the prefixes of the target fragment and the query sequence, the prefix of the target fragment can be successfully used as a key into the hash table to find the group of suffixes of query sequences having the matched prefix. A search of this group, comparing the suffix of the target fragment with all the suffixes contained therein subject to up to two differences, will identify the matching query sequence. Of course, not all matching prefixes will have corresponding matching suffixes. Some matching prefixes may be associated with multiple matching suffixes, because several query sequences match the target sequence fragment subject to the up to two differences.

In hash table methods of exact matching, such as that disclosed by Kim and Kim above, it is sufficient to test for equality between the target fragment and query sequence suffixes. However, for the present purposes a comparison method is desired that is both computationally efficient and capable of detecting the two, or other number of differences between the suffix, or second group derived from the target fragment and the suffix, or second group, derived from the query sequence. This comparison may be carried out by first computing the bitwise exclusive-OR (XOR) of the binary encodings of the two suffixes.

The XOR of two binary numbers contains a 1 at a particular bit position if and only if the two numbers differ at that bit position, so that the number of differences between the suffixes can be counted by counting the positions of the XOR result which contain non-zero bits. Instead of performing this count for each comparison, a look-up table is preferably pre-calculated which contains the number of non-zero bits for all possible values of the XOR result. In this way, two suffixes can be compared by a single XOR operation followed by the retrieval of a value from the look-up table. The XOR of the binary encoding of the two suffixes will contain non zero bits at any base positions where the two suffixes differ. In particular, XOR results containing nonzero bits at two, one or zero base positions identify matches between the target fragment and the corresponding query sequence subject to two, one or zero substitution errors respectively.

## Detailed embodiment

A detailed implementation of the invention, and in particular of the embodiment set out above, will now be described with reference to the figures. Again, this embodiment relates to searching for inexact matches of a large number of query sequences of DNA data in a large target DNA dataset, set out as a plurality of separate chromosome files, and the binary coding scheme discussed above is used. Of course, the method could easily be applied to other data types, and to search for inexact matches subject to different numbers and types of errors.

The implementation described makes use of the insight that the hash functions $f_2$, $f_4$ and $f_6$ described above may be obtained from the functions $f_1$, $f_3$ and $f_5$ by simply exchanging prefixes and suffixes. Advantage is taken of this symmetry by pairing $f_1$ with $f_2$, $f_3$ with $f_4$ and $f_5$ with $f_6$. The hash tables for both members of each pair are generated together, then both hash tables are accessed as the target sequence, set of sequences, or database is scanned. Thus only three scans through the target data are required.

The problem of searching through both strands of the target sequence or database is handled by generating the reverse complement of each query and hashing that as well. Thus on each scan through the target two hash tables are constructed for two complementary hash functions, and each table contains an entry for both the forward and reverse complement of each query sequence.

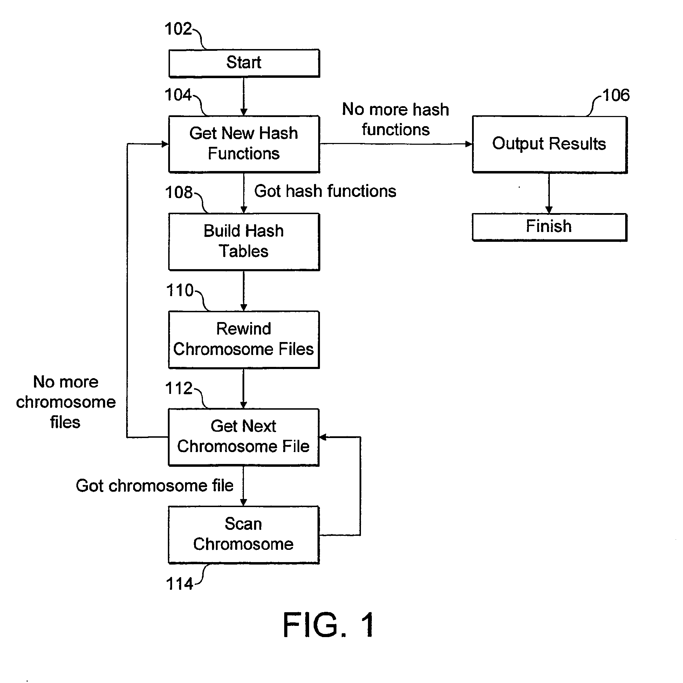

Referring to figure 1, the method is illustrated as a flow diagram. From the start box 102, a new pair of hash functions is selected from $f_1 ... f_6$ in the get new hash functions step 104. If all three pairs of hash functions have already been used then control passes to an output results step 106. Otherwise, two hash tables are built by applying the new pair of hash functions to all of the query sequences and their reverse complements in step 108.

Having built the hash tables, the target sequence data is prepared in the step 110 of rewinding the chromosome files, in preparation for the get next chromosome file step 112 which cycles with the scan chromosome step 114 until all chromosome files have been scanned, and matches between the query sequences and target segments have been recorded. When all chromosome files have been scanned control is returned to the get new hash functions step 104 mentioned above.

When all chromosome files have been scanned using all pairs of hash functions the match results are output in output results step 106. This is implemented by a function that interrogates the relevant data structure, which is **matchstore** discussed below, and outputs the results of the matching process for each query sequence. In the present implementation this output takes the form of one line of ASCII characters for each query sequence, which can be directed to a file. However, the output could equally well be directed to a database.

The get next chromosome file step 112 gets the next of a sequence of chromosome files from a predefined source. In the present implementation a list of file structure directory entries is traversed, but all the chromosome files could equally well be extracted from a database.

The build hash tables step 108 and scan chromosome step 114 will now be described in more detail.

## Build Hash Tables Step

The build hash tables step is implemented using a software function that constructs and populates the data structures outlined below. A C/C++ style notation **A[i]** is used to denote the $i^{th}$ element of an array **A,** with numbering of elements starting at zero.

**MatchStore:** this data structure contains one entry for each query sequence, and is adapted to hold the match results. In the present implementation 8 bytes are used for each entry, and the structure is built as a re-sizeable array, for example using the "vector" template class in the C++ Standard Template Library. Each entry stores the following information:

- a repeat mask flag to show whether the query sequence was repeat masked (discussed below) or, if not repeat masked:
- counts of the number of exact, single error and 2 error matches found (each count has a maximum values of 255 in the present implementation); and, if there is a unique best match:
- position of the unique best match (comprising chromosome, position on chromosome, forward or reverse strand); and
- positions of the differences between the query and its best match (if there are any).

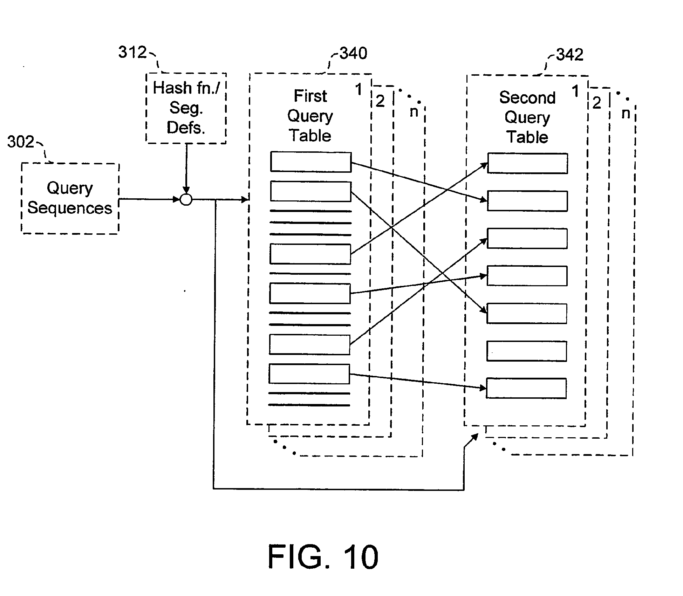

L1: this data structure contains two entries for each non-repeat masked query sequence, and implements the second query tables mentioned above and illustrated in figure 10. Each entry contains the binary encoding of each query sequence suffix (4 bytes in the present implementation). Each entry also contains the entry number of the query sequence in the **MatchStore** structure, and a flag denoting whether this is the forward or reverse strand of the query sequence, together requiring another 4 bytes in the present implementation.

**P1:** this data structure contains one entry for each of the possible values of prefix and implements the hash tables, or first query tables mentioned above and illustrated in figure 10. Noting that the prefix is represented as a binary number having a value of between **0** and **$4^{\text{prefixLength}}$-1** inclusive, **prefixLength** being the number of bases in each prefix, and that there are four different base types, there are **$4^{\text{prefixLength}}$** possible prefixes. Each entry of **P1** acts as a pointer into **L1,** although 32 bit unsigned integers are used in the present implementation. If **X** is the binary encoding of a particular prefix, then the entries of **L1** corresponding to query sequences that have that prefix are held consecutively in **L1,** starting with entry **P1[X]** and stopping at the entry immediately before **P1[X+1].**

**L2** and **P2** are structures which are essentially the same as **L1** and **P1,** except that the roles of the prefix and suffix are reversed, thereby implementing the reverse hash function.

To populate the data structures, the query sequences are read in a predefined order from one or more source files, with some mechanism provided to identify when all the query sequences have been read so that the source files can be rewound. In the present implementation query sequences are read from an ASCII file, but they could equally well be extracted from a database or other storage mechanism.

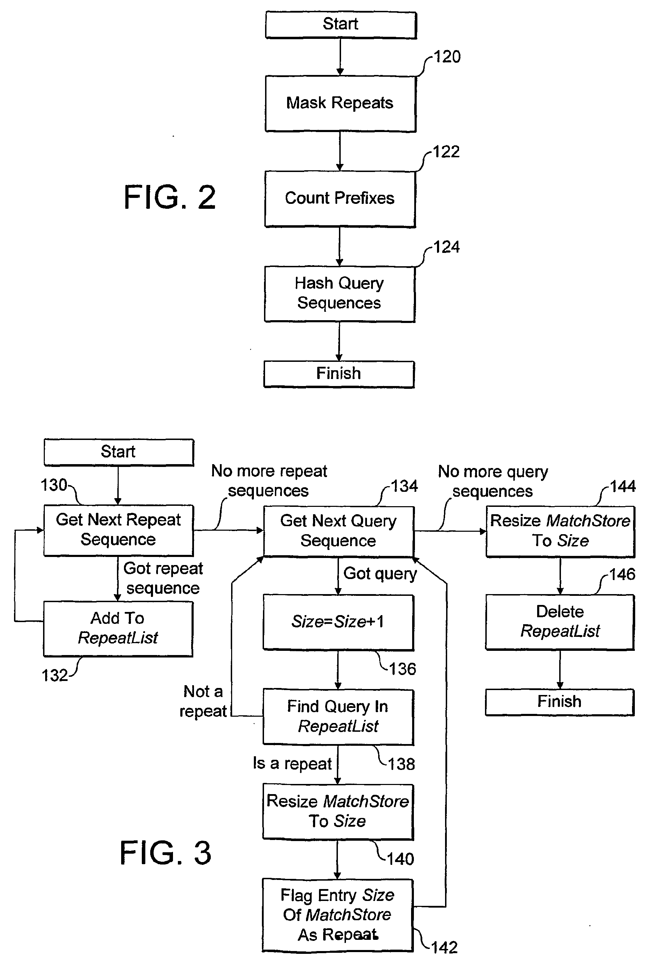

The build hash tables step 108 is broken down into a number of sub-steps as shown in figure 2. A mask repeats step 120 is followed by a count prefixes step 122, then a hash query sequences step 124.

The mask repeats step 120 is illustrated in more detail in figure 3. The aim of this step is to prevent the matching process from being dramatically slowed down or from becoming inefficient by the matching of any one query sequence to a large number of different target sequence segments.

In the mask repeats step 120 a repeat list containing DNA repeat sequences already known to recur, or expected to recur many times in the target data is read. This is illustrated in figure 3 as the cycle between the get next repeat sequence step 130 and the add to repeat list step 132.

When there are no more repeat sequences to be read, processing moves on to reading the query sequences, in the get next query sequence step 134. For every query sequence read, a **size** variable, initially set to zero, is incremented in step 136. If the new query is not found in the repeat list by the subsequent find query in repeat list step 138 then control is returned to the get next query sequence step 134, for the next query sequence to be read. If the new query is found in the repeat list by step 138 then the **MatchStore** structure is resized in step 140 to the size indicated by the **size** variable, and the repeat mask flag of the **MatchStore** entry indexed by the **size** variable is set in step 142. Control is then returned to the get next query sequence step 134.

When there are no more query sequences to be read, **MatchStore** is resized in step 144 to the size indicated by the **size** variable, and the repeat list is deleted in step 146.

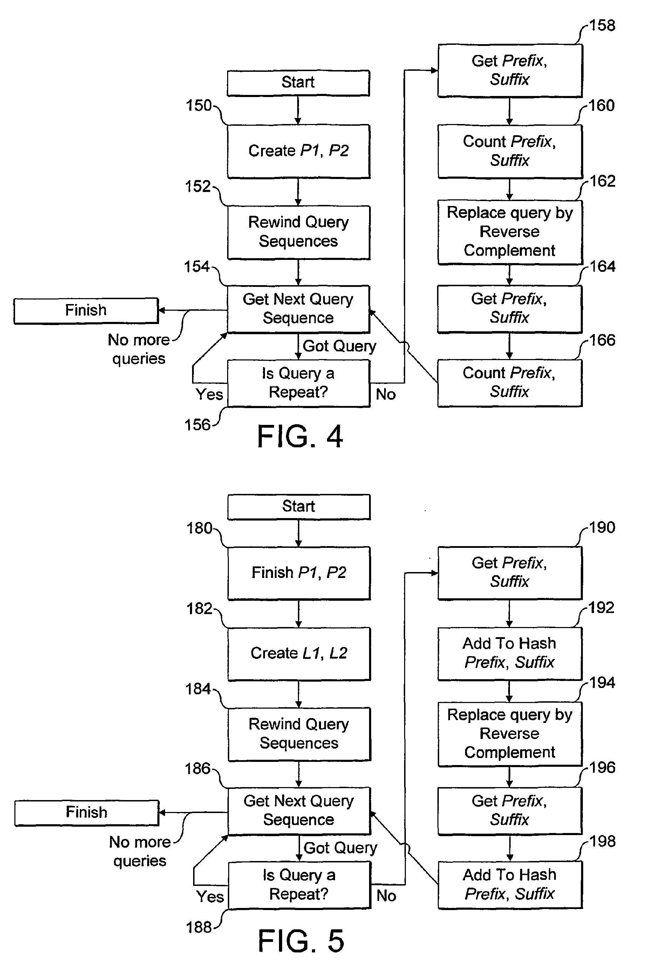

The count prefixes step 122 of figure 2 is illustrated in more detail in figure 4. The aim of the count prefixes step is to count the number of occurrences in the query sequences of each possible prefix into **P1,** and of each possible suffix into **P2.** The structures **P1** and **P2,** as described above, are first created, in step 150. The query sequence file of files are then rewound, in step 152.

The query sequences are then read and processed sequentially by a repeating series of steps beginning with a get next query sequence step 154, until no more queries are available to process. Each query sequence read in step 154 is tested in step 156 to determine if it is found in the repeat list discussed above, by checking the appropriate repeat mask flag of **MatchStore.** If it is found in the repeat list then control is passed back to step 154 to get the next query sequence. Otherwise, the prefix and suffix of the query sequence are derived in step 158, and counted into **P1** and **P2** respectively in step 160 by incrementing the element of **P1** or **P2** indexed by the prefix or suffix. The query sequence is then replaced by its reverse complement in step 162, the prefix and suffix of the reverse complement are derived in step 164 and the newly derived prefix and suffix are counted into **P1** and **P2** in step 166. Control is then returned to the get next query sequence step 154.

The hash query sequences step 124 of figure 2 is illustrated in more detail in figure 5. The aim of this step is to populate the **L1** and **L2** structures. The first sub-step is the finish P1 P2 step 180 in which **P1** and **P2** are traversed, and converted into cumulative sums. The last elements of **P1** and **P2** then indicate the total number of non repeat masked query sequences, including both forward and reverse complements, and hence the required sizes for **L1** and **L2**. These sizes are used in the subsequent step 182 of creating the **L1** and **L2** structures, following which the one or more query sequence files are rewound in step 184, ready to be read again.

The query sequences are then read and processed sequentially by a repeating series of steps beginning with a get next query sequence step 186, until no more queries are available to process. Each query sequence read in step 186 is tested in step 188 to determine if it is found in the repeat list discussed above, by checking the appropriate repeat mask flag of **MatchStore.** If it is found in the repeat list then control is passed back to step 186 to get the next query sequence. Otherwise, the prefix and suffix of the query sequence are derived in step 190, and written into the **L2** and **L1** structures respectively in step 192. The query sequence is then replaced by its reverse complement in step 194, the prefix and suffix of the reverse complement are derived in step 196 and the newly derived prefix and suffix are written into **L2** and **L1** in step 198. Control is then returned to the get next query sequence step 186.

### Scan chromosome step

In this step a chromosome sequence is scanned, base by base. At each base position, where possible, a target sequence fragment of the same length as the query sequences is extracted. This may not always be possible: if the target fragment contains a code indicating an unknown or ambiguous base then the fragment is ignored, and the process skips to the next useable fragment.

Each chromosome file could be an ASCII file, but in the present implementation one or more binary files using the 2-bits-per-base binary encoding discussed above provide the target data. Ambiguity codes are handled by maintaining a separate list of "valid" regions free from ambiguity codes, each region being defined by a start and a stop position.

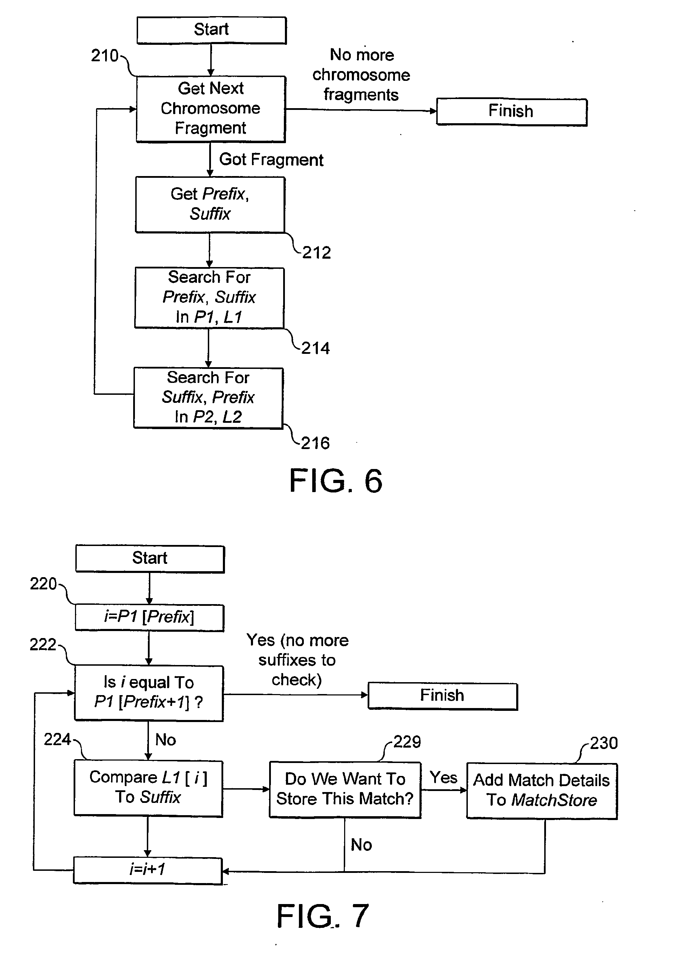

The scan chromosome step is illustrated in more detail in figure 6. The get next chromosome fragment step 210 obtains the next target fragment available from within the valid regions mentioned above. The prefix and suffix of the target fragment are then obtained using the current hash functions in step 212. The target fragment prefix and suffix are sought in **P1** and **L1** respectively in step 214, and the suffix and prefix are sought in **P2** and **L2** respectively in step 216. Control is then passed back to the get next chromosome fragment step 210.

When no more chromosome fragments are available the scan chromosome task 114 of figure 1 is complete.

The step 214 of seeking the current target fragment prefix and suffix in **P1** and **L1** is further illustrated in figure 7. In step 220 a count variable **i** is set to the value of **P1** corresponding to the present target fragment prefix. Variable **i** is then used to count through the entries of **L1** corresponding to all the queries having the same prefix, by means of increment step 226 until **i** is equal to the value of **P1** corresponding to the next possible prefix, as determined by test step 222, at which point the step 214 of figure 6 is complete.

For each value of count variable i, the query sequence suffix held in L1[i] is compared, in step 224, to the present target fragment suffix. In this way, the target suffix is compared with the suffixes of all query sequences that have the same prefix as the target fragment. As discussed above, this is achieved by taking the bitwise exclusive-OR of the binary encoding of the target suffix with the binary encoding of each query suffix, and then using the result to index into a lookup table to find the number of mismatches and their positions.

Comparisons revealing more than two mismatches are of no interest in the present implementation. However, it will be recalled that matches having mismatch errors in two distinct segments will be found using only one of the six hash functions, those having mismatch errors in only one segment will be found using two of the hash functions, and exact matches will be found using all six hash functions. For this reason, the results of comparisons revealing mismatches in two distinct segments are always stored, but by choosing to ignore other types of matches on certain passes it is ensured that an exact or inexact match is registered only once.

The decision whether or not to store a match found using comparison step 224 is made in store decision step 229. If a positive decision is made, the comparison results are stored in the **MatchStore** structure in step 230. In either case, control is returned to test step 222 by way of increment step 226.

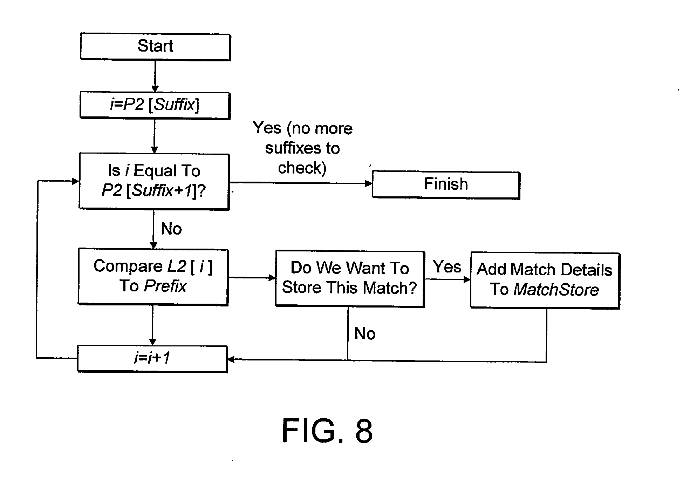

The step 216 of seeking the current target fragment suffix and prefix in **P2** and **L2** respectively is further illustrated in figure 8. It will be seen that the steps of figure 8 are identical to those of figure 7, except that **P1** is replaced by **P2, L1** by **L2,** "prefix" by "suffix", and "suffix" by "prefix", such that the roles of the suffix and prefix are reversed and the complementary hash function implemented.

**Apparatus**

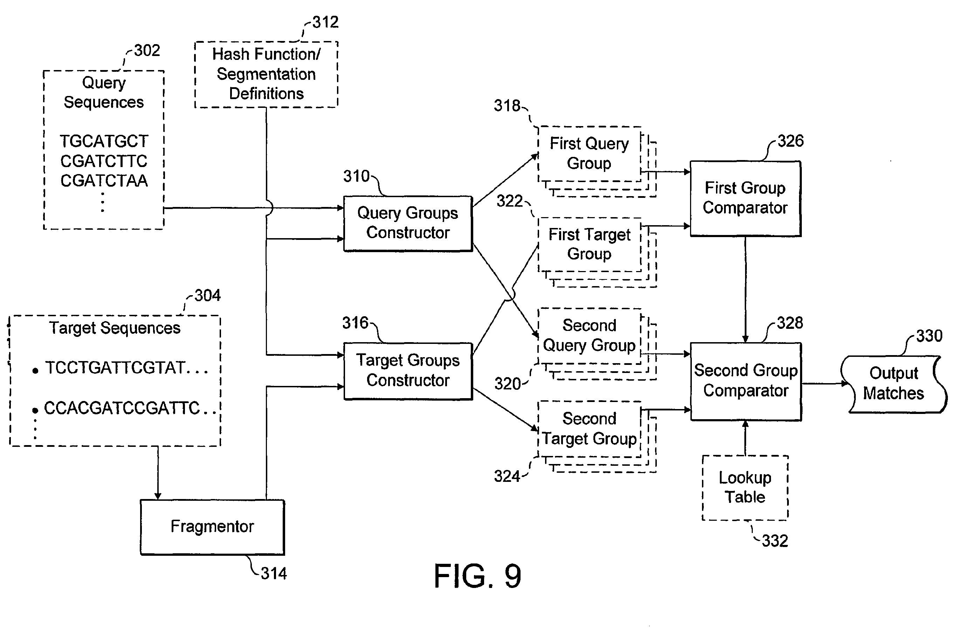

Computer apparatus for putting the above described methods into effect is illustrated in figure 9. Query sequences 302 and target sequences 304 are stored in volatile or non volatile memory, for example in a database or on a hard disk drive. A query groups constructor element 310 is provided by software running on the computer, and constructs query groups fromm segments of the query sequences, as determined by data or program elements 312 representing the hash functions, or segmentation distributions to be used.

A fragmentor element 314 takes target sequence data 304 and from it forms target sequence fragments of the same length as the query sequences. The fragmentor avoids target sequences containing ambiguity codes by referring to a list of valid regions.

Target sequence fragments are passed to the target groups constructor 316, which constructs target groups of segments of the target fragments, also under the control of the data or program elements 312 defining the hash functions or segment distributions to be used.

The query groups constructor 310 forms pairs of first and second query groups 318, 320 of segments for each query sequence and each hash function. The target groups constructor 316 forms corresponding pairs of first and second target groups 322, 324 of target fragment segments, for each target sequence fragment and each hash function.

Each first target group 322 is compared with each first query group 318 by means of a first comparator mechanism 326, which is implemented using the hash table methodology discussed above, and illustrated as the first query table 340 in figure 10. An exact match between a first query group 318 and a first target group 322 indicates a possible match between the corresponding full query sequence and target fragment. This possible match is signalled to a second group comparator mechanism 328 which compares the associated second query and target groups, making use of the second query table 342 illustrated in figure 10. If the second groups match, subject to the maximum number of mismatches allowed according to the hash functions 312 used, then a match result is output by output element 330. The second group comparison is preferably implemented, as discussed above, by an XOR operation, the result of which is used as an index into a lookup table 332.

Some data structures formed in memory by the apparatus of figure 9 and the method described in connection with figures 1 to 8 are shown in figure 10. Query sequences 302 and hash function or segmentation definitions 312 are jointly used to create a set of first query tables 340. There is a query table for each hash function, each table being indexed by the possible values of first query groups of query sequence segments which could be produced using the corresponding hash function. The elements of each first query table, or hash table, provide references into entries of a corresponding second query table 342. There is a second query table for each hash function. Each second query table contains the suffixes, or second query groups of segments actually produced using the corresponding hash function.

Using the data structures illustrated in figure 10, a target fragment is processed using a hash function to form a prefix, or first query group, and the corresponding entry in the first query table 340 for the same hash function is used to find the entries in the appropriate second query table 342 containing query sequence suffixes which might confirm an exact or in exact match.

Of course, the query tables for the different hash functions do not need to coexist in memory at the same time, although simultaneous construction and/or use of tables corresponding to a complementary pair of hash functions may be advantageous, as already discussed.

**Specific implementation**

The described method and apparatus were used, in particular, to search for exact or inexact matches between large numbers of short DNA query sequences and target sequences in a database containing relatively small numbers of large DNA sequences structured in files by chromosome. In one implementation, a batch of about 12 million query sequences of between 16 and 32 bases in length were scanned against a target database of build 33 of the human genome. This version of the human genome comprised files for each of the 22 autosomal chromosomes and each of the 2 sex chromosomes, containing about 3 billion bases in total. This was carried out on an Intel Pentium desktop computer with a microprocessor clock speed of 2GHz and 1GByte of RAM, running the well known RedHat Linux 7.2 operating system. The method was implemented using software code written in C++ and compiled using the well known GNU C/C++ compiler.

**Variations and extensions**

The methods discussed in detail above are a special case of a more general method that seeks matches subject to **m** differences by splitting query sequences into n fragments, where **n > m.** Generally, $^{n}C_{m} = n! / (m! (n-m)!)$ passes through the target databases will be used, each time carrying out the hash table scheme using a different one of the $^{n}C_{m}$ ways of choosing **n** minus **m** fragments for exact matching the prefixes and **m** fragments for inexact matching of the suffixes. In the above detailed examples, setting **n = 2m** allows hash functions to be paired, leading to greater computational efficiency.

By way of example, if **n = 2** and **m = 1,** then the method will find inexact matches subject to a single mismatch using two hash functions, which are trivial in that they merely define a prefix and suffix, and a corresponding suffix and prefix. If **n = 3** and **m = 2,** then the method will find inexact matches subject to two mismatches using three hash functions, but may be much less efficient than using **n = 4** and **m = 2** because the sizes of the hash tables stored in the **P** data structures will be rather small, and the number of query suffixes for any given query prefix rather large.

If **n = 6** and **m = 3,** all three-error matches can be found in 20 passes through the target data, or in 10 passes if complementary hash functions are paired together as already described.

For a given number of allowable errors to accommodate an inexact match, i.e. **m,** a higher value of **n** would be expected to make each pass faster, but there will be more passes to complete. For greater **n,** the lookup tables **P1** and **P2** also rapidly become very large, and may not fit into fast memory. To fine tune the method to cope with this, **P1** and **P2** may contain only most significant bits of the prefixes, with the least significant bits being accommodated in **L1** and **L2.** However, lookup of suffix information then requires an additional binary search phase following the initial table lookup. This scheme has been tried for query sequence lengths greater than 26 bases, and it works well without being detrimental to the speed of the method.

The number of allowable errors in an inexact match **m** is likely to be governed by the application to which the method is being put, while the value of **n** to optimize the method will be very hardware dependent, in particular being governed by the amount of fast memory available to accommodate the required data structures.

**Claims**

1. A computer implemented method of searching for a plurality of query sequences in a set of target sequence fragments, allowing for mismatches at up to **n** sequence positions, said query sequences and target sequence fragments comprising biochemical sequence data, comprising the steps of:

    a) logically dividing each query sequence and each target sequence fragment into at least **n+1** segments;
    b) for each query sequence, constructing a first and a second query group by distributing the query sequence segments there between such that at least n segments are contained in the second query group;

c) constructing from each target fragment a first target group having the same segment distribution as the first query group;

d) for each query sequence, comparing said first query group with each first target group to identify potential matching target fragments;

e) repeating steps (b) to (d) using different distributions of segments between said query groups such that all of the mismatches at **n** unknown sequence positions will lie in the second query group for at least one of the distributions; and

f) for each potential matching target fragment identified in step (d), constructing a second target group having the same segment distribution as the second query group of the potentially matching query sequence and comparing said second query group with said second target group to identify a match, allowing for mismatches in up to **n** sequence positions.

2. The method of claim 1 wherein steps (b) to (d) are repeated using all distinct distributions of segments between said query groups.

3. The method of claim 5 wherein the comparing of step (f) is carried out by applying an exclusive OR operation to binary representations of the second query group and second target group.

4. The method of claim 3 wherein the result of said exclusive OR operation is analysed using a lookup table.

5. The method of any preceding claim further comprising, for each distinct distribution of query sequence segments, constructing a first query table indexed by possible values of the first query groups, the entries in the first query table providing access to each second query group by using as an index the value of a corresponding first query group.

6. The method of claim 5 further comprising, for each distinct distribution of query sequence segments, constructing a second query table providing access to each second query group, the entries in the first query table providing references to appropriate entries in the second query table.

7. The method of claim 5 or 6 further comprising the step of, for each first target group constructed in step (c), implementing step (d) by using said first target group to form an index into said first query table.

8. The method of any of claims 5 to 7 wherein, if two distinct distributions of query sequence segments are such that the first query group of one distribution is the same as the second query group of the other distribution, said query tables for both of the distinct distributions are constructed and/or used concurrently.

9. The method of any preceding claim wherein said target sequence fragments comprise overlapping fragments of one or more target sequences.

10. The method of any preceding claim wherein the query sequences are nucleic acid sequences each between about 16 and 32 bases in length, and the target sequence fragments are fragments of genome data.

11. The method of claim 10 wherein said query sequences and target sequence fragments are binary encoded.

12. The method of any preceding claim wherein n is at least two.

13. The method of any preceding claim wherein n is two and each query sequence and target fragment is divided into four segments.

14. The method of any preceding claim wherein said query sequences and target sequence fragments are logically divided into an even number of segments, and the segments are distributed in equal numbers between the first and second query and target groups.

15. A computer program product carrying computer program code comprising elements adapted to carry out the method of any of claims 1 to 14 when executed on a computer.

16. A computer readable medium comprising computer program code adapted to carry out the method steps of any of claims 1 to 14 when executed on a computer.

17. Computer apparatus comprising a memory, for searching for a plurality of query sequences (302) in a set of target sequence fragments, allowing for mismatches at up to **n** sequence positions, said query sequences and target sequence fragments comprising biochemical sequence data, comprising:

a query groups constructor (310) adapted to construct in the memory, for each query sequence, first and second query groups by logically dividing each query sequence into at least **n+1** segments and distributing the query sequence segments between the first and second query groups in one or more ways such that at least n segments are contained in each second query group;

a target groups constructor (316) adapted to construct in the memory, for each target sequence fragment, one or more first target groups having segment distributions corresponding to the first query groups;

a first group comparator (326) adapted, for each query sequence, to compare said one or more first query groups with corresponding ones of said one or more first target groups and to thereby output a result identifying potentially matching query sequences and target fragments, wherein the query groups constructor and target groups constructor are adapted to construct, for each query sequence and target fragment respectively, a plurality of groups having different distributions of segments between said first and second groups such that all of the mismatches at **n** unknown sequence positions will lie in the second query group for at least one of the distributions, and

wherein the target groups constructor is adapted to construct, at least for each target sequence fragment identified by the first group comparator as a potentially matching target fragment, one or more second target groups having segment distributions corresponding to the second query groups, the apparatus further comprising a second group comparator (328) adapted, for each identified potentially matching query group and target fragment, to compare the corresponding second query group and second target group to identify a match allowing for mismatches in up to **n** sequence positions.

18. The apparatus of claim 17, wherein the query groups constructor and target groups constructor are adapted to construct, for each query sequence and target fragment respectively, a plurality of groups having all distinct distributions of segments between said first and second groups.

19. The apparatus of claim 17 or 18, further adapted, for each distinct distribution of query sequence segments, to construct in the memory a first query table indexed by possible values of the first query groups, the entries in the first query table providing access to each second query group by using as an index the value of a corresponding first query group.

20. The apparatus of claim 19 further adapted, for each distinct distribution of query sequence segments, to construct in the memory a second query table providing access to each second query group, the entries in the first query table providing references to appropriate entries in the second query table.

21. The apparatus of any of claims 17 to 20 wherein n is at least two.

22. The apparatus of claim 21 where **n** is two and the query groups constructor and target groups constructor are each adapted to divide each query sequence and target sequence fragment into four segments.

23. The apparatus of any of claims 17 - 22 wherein the query sequences are nucleic acid sequences each between about 16 and 32 bases in length, and the target sequence fragments are fragments of genome data.

24. The apparatus of claim 17 further adapted to carry out the method steps of any of claims 1 to 14.

**Patentansprüche**

1. Computerimplementiertes Verfahren zur Suche nach einer Vielzahl von Abfragesequenzen in einer Gruppe von Zielsequenzfragmenten, welches Nichtübereinstimmungen an bis zu n Sequenzpositionen ermöglicht bzw. einkalkuliert, wobei die Abfragesequenzen und Zielsequenzfragmente biochemische Sequenzdaten aufweisen, **dadurch gekennzeichnet, dass** das Verfahren die folgenden Schritte aufweist:

a) logische Teilung einer jeden Abfragesequenz und eines jeden Zielsequenzfragments in mindestens n+1 Segmente;
b) für jede Abfragesequenz, Konstruktion einer ersten und einer zweiten Abfragegruppe durch Verteilung der Abfragesequenzfragmente zwischen bzw. unter den beiden, so dass zumindest n Segmente in der zweiten Abfragegruppe enthalten sind;
c) Konstruktion einer ersten Zielgruppe aus jedem Zielfragment, wobei die erste Zielgruppe dieselbe Segmentverteilung wie die erste Abfragegruppe aufweist;
d) für jede Abfragesequenz, Vergleich der ersten Abfragegruppe mit jeder ersten Zielgruppe zur Identifikation potentieller übereinstimmender Zielfragmente;
e) Wiederholung der Schritte (b) bis (d) unter Verwendung unterschiedlicher Verteilungen von Segmenten zwischen bzw. unter den Abfragegruppen, so dass alle Nichtübereinstimmungen an n unbekannten Sequenzpositionen in der zweiten Abfragegruppe für mindestens eine der Verteilungen liegen; und
f) für jedes in Schritt d) identifizierte potentielle übereinstimmende Zielfragment, Konstruktion einer zweiten Zielgruppe, welche dieselbe Segmentverteilung wie die zweite Abfragegruppe der potentiell übereinstimmenden Abfragesequenz aufweist, und Vergleich der zweiten Abfragegruppe mit der zweiten Zielgruppe zur Identifikation einer Übereinstimmung, welche Nichtübereinstimmungen in bis zu n Sequenzpositionen ermöglicht bzw. einkalkuliert.

2. Verfahren nach Anspruch 1, **dadurch gekennzeichnet, dass** die Schritte (b) bis (d) wiederholt werden, indem alle eindeutigen Segmentverteilungen unter bzw. zwischen den Abfragegruppen verwendet werden.

3. Verfahren nach Anspruch 5, **dadurch gekennzeichnet, dass** der Vergleich von Schritt (f) ausgeführt wird, indem eine exklusive ODER-Funktion bzw. Antivalenz an binäre Darstellungen der zweiten Abfragegruppe und der zweiten Zielgruppe angelegt wird.

4. Verfahren nach Anspruch 3, **dadurch gekennzeichnet, dass** das Ergebnis der exklusiven ODER-Funktion analysiert wird, indem eine Nachschlagetabelle verwendet wird.

5. Verfahren nach einem der vorhergehenden Ansprüche, welches des Weiteren den folgenden Schritt aufweist: für jede eindeutige Verteilung von Abfragesequenzsegmenten, Konstruktion einer ersten Abfragetabelle, welche durch mögliche Werte der ersten Abfragegruppen indiziert ist, wobei die Einträge in der ersten Abfragetabelle Zugriff auf jede zweiten Abfragegruppe bereitstellen, indem der Wert einer entsprechenden ersten Abfragegruppe als Index verwendet wird.

6. Verfahren nach Anspruch 5, welches des Weiteren den folgenden Schritt aufweist: für jede eindeutige Verteilung von Abfragesequenzsegmenten, Konstruktion einer zweiten Abfragetabelle, welche Zugriff auf jede zweite Abfragegruppe bereitstellt, wobei die Einträge in der ersten Abfragetabelle Verweise auf geeignete Einträge in der zweiten Abfragetabelle liefern.

7. Verfahren nach Anspruch 5 oder 6, welches des Weiteren den folgenden Schritt aufweist: für jede in Schritt (c) konstruierte erste Zielgruppe, Implementierung von Schritt (d), indem die erste Zielgruppe zur Bildung eines Index in der ersten Abfragetabelle verwendet wird.

8. Verfahren nach einem der Ansprüche 5 bis 7, **dadurch gekennzeichnet, dass** wenn zwei eindeutige Verteilungen von Abfragesequenzsegmenten derart ausgelegt sind, dass die erste Abfragegruppe einer Verteilung identisch mit der zweiten Abfragegruppe der anderen Verteilung ist, die Abfragetabellen für beide eindeutigen Verteilungen gleichzeitig konstruiert und/oder verwendet werden.

9. Verfahren nach einem beliebigen vorhergehenden Anspruch, **dadurch gekennzeichnet, dass** die Zielsequenzfragmente sich überlappende Fragmente einer oder mehrerer Zielsequenzen aufweisen.

10. Verfahren nach einem beliebigen vorhergehenden Anspruch, **dadurch gekennzeichnet, dass** die Abfragesequenzen Nukleinsäuresequenzen sind, welche jeweils zwischen ungefähr 16 und 32 Basen Länge aufweisen, und dass die Zielsequenzfragmente Fragmente von Genomdaten sind.

11. Verfahren nach Anspruch 10, **dadurch gekennzeichnet, dass** die Abfragesequenzen und die Zielsequenzfragmente binär codiert werden.

12. Verfahren nach einem beliebigen vorhergehenden Anspruch, **dadurch gekennzeichnet, dass** n mindestens zwei ist.

13. Verfahren nach einem beliebigen vorhergehenden Anspruch, **dadurch gekennzeichnet, dass** n zwei ist und jede Abfragesequenz und jedes Zielfragment in vier Segmente unterteilt wird.

14. Verfahren nach einem beliebigen vorhergehenden Anspruch, **dadurch gekennzeichnet, dass** die Abfragesequenzen und Zielsequenzfragmente logisch in eine gerade Anzahl von Segmenten geteilt werden, und dass die Segmente in gleicher Anzahl zwischen bzw. unter den ersten und zweiten Abfrage- und Zielgruppen verteilt werden.

15. Computerprogrammprodukt, welches einen Computerprogrammcode mit sich führt, welcher Elemente umfasst, welche zur Ausführung des Verfahrens nach einem der Ansprüche 1 bis 14 ausgelegt sind, wenn er auf einem Computer ausgeführt wird.

16. Computerlesbares Medium, welches einen Computerprogrammcode aufweist, welcher derart ausgelegt ist, dass er die Verfahrensschritte nach jedem der Ansprüche 1 bis 14 verrichtet, wenn er auf einem Computer ausgeführt oder angewendet wird.

17. Computervorrichtung mit einem Speicher, zur Suche nach einer Vielzahl von Abfragesequenzen (302) in einer Gruppe von Zielsequenzfragmenten, welche Nichtübereinstimmungen an bis zu n Sequenzpositionen ermöglichen bzw. einkalkulieren, wobei die Abfragesequenzen und Zielsequenzfragmente biochemische Sequenzdaten aufweisen, wobei die Computervorrichtung Folgendes aufweist:

einen Abfragegruppen-Konstruktor (310), welcher derart ausgelegt ist, dass er in dem Speicher für jede Abfragesequenz erste und zweite Abfragegruppen konstruiert, indem er jede Abfragesequenz logisch in mindestens n+1 Segmente teilt und die Abfragesequenzsegmente auf eine oder mehrere Arten zwischen bzw. unter den ersten und zweiten Abfragegruppen derart verteilt, dass mindestens n Segmente in jeder zweiten Abfragegruppe enthalten sind;

einen Zielgruppen-Konstruktor (316), welcher derart ausgelegt ist, dass er in dem Speicher für jedes Zielsequenzfragment eine oder mehrere erste Zielgruppen konstruiert, welche den ersten Abfragegruppen entsprechende Segmentverteilungen aufweisen ;

einen ersten Gruppenvergleicher (326), welcher derart ausgelegt ist, dass er für jede Abfragesequenz diese eine oder mehrere erste Abfragegruppen mit entsprechenden der einen oder mehreren ersten Zielgruppen vergleicht und auf diese Weise ein Ergebnis ausgibt, welches potentiell übereinstimmende Abfragesequenzen und Zielfragmente identifiziert,

**dadurch gekennzeichnet, dass** der Abfragegruppen-Konstruktor und der Zielgruppen-Konstruktor derart ausgelegt sind, dass sie für jede Abfragesequenz bzw. jedes Zielfragment eine Vielzahl von Gruppen konstruieren, welche unterschiedliche Verteilungen von Segmenten zwischen bzw. unter den ersten und zweiten Gruppen aufweisen, so dass alle Nichtübereinstimmungen an n unbekannten Sequenzpositionen in der zweiten Abfragegruppe für mindstens eine der Verteilungen liegen, und

**dadurch gekennzeichnet, dass** der Zielgruppen-Konstruktor derart ausgelegt ist, dass er zumindest für jedes Zielsequenzfragment, welches von dem ersten Gruppenvergleicher als ein potentiell übereinstimmendes Zielfragment identifiziert wird, eine oder mehrere zweite Zielgruppen konstruiert, welche den zweiten Abfragegruppen entsprechende Segmentverteilungen aufweisen, wobei die Vorrichtung des Weiteren einen zweiten Gruppenvergleicher (328) aufweist, welcher derart ausgelegt ist, dass er für jede identifizierte potentiell übereinstimmende Abfragegruppe und jedes Zielfragment die entsprechende zweite Abfragegruppe und zweite Zielgruppe zur Indentifizierung einer Übereinstimmung vergleicht, welche Nichtübereinstimmungen in bis zu n Sequenzpositionen zulässt.

18. Vorrichtung nach Anspruch 17, **dadurch gekennzeichnet, dass** der Abfragegruppen-Konstruktor und der Zielgruppen-Konstruktur derart ausgelegt sind, dass sie für jede Abfragesequenz bzw. jedes Zielfragment eine Vielzahl von Gruppen konstruieren, welche alle eindeutige Verteilungen von Segmenten unter bzw. zwischen den ersten und zweiten Gruppen aufweisen.

19. Vorrichtung nach Anspruch 17 oder 18, welche des Weiteren derart ausgelegt ist, dass sie für jede eindeutige Verteilung von Abfragesequenzsegmenten in dem Speicher eine erste Abfragetabelle konstruiert, welche durch mögliche Werte der ersten Abfragegruppen indiziert ist, wobei die Einträge in der ersten Abfragetabelle Zugriff auf jede zweite Abfragegruppe bereitstellen, indem der Wert einer entsprechenden ersten Abfragegruppe als Index verwendet wird.

20. Vorrichtung nach Anspruch 19, welche des Weiteren derart ausgelegt ist, dass sie für jede eindeutige Verteilung von Abfragesequenzsegmenten in dem Speicher eine zweite Abfragetabelle konstruiert, welche Zugriff auf jede zweite Abfragegruppe bereitstellt, wobei die Einträge in der ersten Abfragetabelle Verweise auf geeignete Einträge in der zweiten Abfragetabelle bereitstellen.

21. Vorrichtung nach einem der Ansprüche 17 bis 20, **dadurch gekennzeichnet, dass** n zumindest zwei ist.

22. Vorrichtung nach Anspruch 21, **dadurch gekennzeichnet, dass** n zwei ist und der Abfragegruppen-Konstruktor und der Zielgruppen-Konstruktor jeweils derart ausgelegt sind, dass sie jede Abfragesequenz bzw. jedes Zielsequenzfragment in vier Segmente teilen.

23. Vorrichtung nach einem der Ansprüche 17 bis 22, **dadurch gekennzeichnet, dass** die Abfragesequenzen Nukleinsäuresequenzen jeweils zwischen ungefähr 16 und 32 Basen Länge sind, und dass die Zielsequenzfragmente Fragmente von Genomdaten sind.

24. Vorrichtung nach Anspruch 17, welche des Weiteren so ausgelegt ist, dass sie die Verfahrensschritte nach jedem der Ansprüche 1 bis 14 ausführt.

## Revendications

1. Procédé mis en oeuvre sur un ordinateur pour rechercher une pluralité de séquences d'interrogation dans un ensemble de fragments de séquences cibles, permettant des absences de correspondance jusqu'à n positions de séquence, lesdites séquences d'interrogation et les fragments de séquences cibles comprenant des données de séquences biochimiques, comprenant les étapes consistant à:

a) diviser logiquement chaque séquence d'interrogation et chaque fragment de séquence cible en au moins n+1 segments;

b) pour chaque séquence d'interrogation, construire des premier et deuxième groupes d'interrogation en répartissant les segments de séquence d'interrogation entre eux de sorte qu'au moins n segments soient contenus dans le deuxième groupe d'interrogation;

c) construire, à partir de chaque fragment cible, un premier groupe cible ayant la même répartition de segments que le premier groupe d'interrogation;

d) pour chaque séquence d'interrogation, comparer ledit premier groupe d'interrogation avec chaque premier groupe cible pour identifier des fragments cibles potentiellement correspondants;

e) répéter les étapes (b) à (d) en utilisant différentes répartitions de segments entre lesdits groupes d'interrogation de sorte que toutes les absences de correspondance à n positions de séquence inconnues se trouveront dans le deuxième groupe d'interrogation pour au moins l'une des répartitions; et

f) pour chaque fragment cible potentiellement correspondant identifié à l'étape (d), construire un deuxième groupe cible ayant la même répartition de segments que le deuxième groupe d'interrogation de la séquence d'interrogation potentiellement correspondante et comparer ledit deuxième groupe d'interrogation audit deuxième groupe cible pour identifier une correspondance, permettant des absences de correspondance jusqu'à n positions de séquence.

2. Procédé selon la revendication 1, dans lequel les étapes (b) à (d) sont répétées en utilisant toutes les répartitions distinctes de segments entre lesdits groupes d'interrogation.

3. Procédé selon la revendication 5, dans lequel la comparaison de l'étape (f) est effectuée en appliquant une opération OU exclusif aux représentations binaires du deuxième groupe d'interrogation et du deuxième groupe cible.

4. Procédé selon la revendication 3, dans lequel le résultat de ladite opération OU exclusif est analysé en utilisant une table de correspondance.

5. Procédé selon l'une quelconque des revendications précédentes, comprenant en outre, pour chaque répartition distincte de segments de séquence d'interrogation, la construction d'une première table d'interrogation indexée par des valeurs possibles des premiers groupes d'interrogation, les entrées dans la première table d'interrogation fournissant un accès à chaque deuxième groupe d'interrogation en utilisant en tant qu'index la valeur d'un premier groupe d'interrogation correspondant.

6. Procédé selon la revendication 5, comprenant en outre, pour chaque répartition distincte de segments de séquence d'interrogation, la construction d'une deuxième table d'interrogation fournissant un accès à chaque deuxième groupe d'interrogation, les entrées dans la première table d'interrogation fournissant des références vers des entrées appropriées dans la deuxième table d'interrogation.

7. Procédé selon la revendication 5 ou 6, comprenant en outre l'étape, pour chaque premier groupe cible construit à l'étape (c), consistant à effectuer l'étape (d) en utilisant ledit premier groupe cible pour former un index dans ladite première table d'interrogation.

8. Procédé selon l'une quelconque des revendications 5 à 7, dans lequel, si deux répartitions distinctes de segments de séquence d'interrogation sont telles que le premier groupe d'interrogation d'une répartition est identique au deuxième groupe d'interrogation de l'autre répartition, lesdites tables d'interrogation pour les deux répartitions distinctes sont construites et/ou utilisées simultanément.

9. Procédé selon l'une quelconque des revendications précédentes, dans lequel lesdits fragments de séquences cibles comprennent des fragments superposés d'une ou de plusieurs séquences cibles.

10. Procédé selon l'une quelconque des revendications précédentes, dans lequel les séquences d'interrogation sont des séquences d'acide nucléique, chacune d'une longueur entre environ 16 et 32 bases, et les fragments de séquences cibles sont des fragments de données de génome.

11. Procédé selon la revendication 10, dans lequel lesdites séquences d'interrogation et les fragments de séquences cibles sont encodés de manière binaire.

12. Procédé selon l'une quelconque des revendications précédentes, dans lequel n est au moins égal à deux.

13. Procédé selon l'une quelconque des revendications précédentes, dans lequel n est égal à deux et chaque séquence d'interrogation et fragment cible est divisé en quatre segments.

14. Procédé selon l'une quelconque des revendications précédentes, dans lequel lesdites séquences d'interrogation et les fragments de séquences cibles sont divisés logiquement en un nombre pair de segments, et les segments sont répartis en nombres égaux entre les premier et deuxième groupes d'interrogation et cibles.

15. Produit-programme informatique comportant un code de programme informatique comprenant des éléments adaptés pour mettre en oeuvre le procédé selon l'une quelconque des revendications 1 à 14 lorsqu'il est exécuté sur un ordinateur.

16. Support pouvant être lu par un ordinateur comprenant un code de programme informatique adapté pour mettre en oeuvre les étapes de procédé selon l'une quelconque des revendications 1 à 14 lorsqu'il est exécuté sur un ordinateur.

17. Dispositif informatique comprenant une mémoire, pour rechercher une pluralité de séquences d'interrogation (302) dans un ensemble de fragments de séquences cibles, permettant des absences de correspondance jusqu'à n positions de séquence, lesdites séquences d'interrogation et les fragments de séquences cibles comprenant des données de séquences biochimiques, comprenant:

un constructeur de groupes d'interrogation (310) adapté pour construire dans la mémoire, pour chaque séquence d'interrogation, des premier et deuxième groupes d'interrogation en divisant logiquement chaque séquence d'interrogation en au moins n+1 segments et en répartissant les segments de séquence d'interrogation entre les premier et deuxième groupes d'interrogation d'une ou de plusieurs manières de sorte qu'au moins n segments soient contenus dans chaque deuxième groupe d'interrogation; un constructeur de groupes cibles (316) adapté pour construire dans la mémoire, pour chaque fragment de séquence cible, un ou plusieurs premiers groupes cibles ayant des répartitions de segments correspondant aux premiers groupes d'interrogation; un premier comparateur de groupes (326) adapté pour comparer, pour chaque séquence d'interrogation, lesdits un ou plusieurs premiers groupes d'interrogation avec ceux correspondants desdits un ou plusieurs premiers groupes cibles et pour délivrer de ce fait un résultat identifiant des séquences d'interrogation et des fragments cibles potentiellement correspondants,

dans lequel le constructeur de groupes d'interrogation et le constructeur de groupes cibles sont adaptés pour construire, pour chaque séquence d'interrogation et fragment cible respectivement, une pluralité de groupes ayant différentes répartitions de segments entre lesdits premier et deuxième groupes de sorte que toutes les absences de correspondance à n positions de séquence inconnues se trouveront dans le deuxième groupe d'interrogation pour au moins l'une des répartitions, et
dans lequel le constructeur de groupes cibles est adapté pour construire, au moins pour chaque fragment de séquence cible identifié par le premier comparateur de groupes comme étant un fragment cible potentiellement correspondant, un ou plusieurs deuxièmes groupes cibles ayant des répartitions de segments correspondant aux deuxièmes groupes d'interrogation, le dispositif comprenant en outre un deuxième comparateur de groupes (328) adapté pour comparer, pour chaque groupe d'interrogation et fragment cible potentiellement correspondants identifiés, le deuxième groupe d'interrogation et le deuxième groupe cible correspondants pour identifier une correspondance permettant des absences de correspondance jusqu'à n positions de séquence.

18. Dispositif selon la revendication 17, dans lequel le constructeur de groupes d'interrogation et le constructeur de groupes cibles sont adaptés pour construire, pour chaque séquence d'interrogation et fragment cible respectivement, une pluralité de groupes comportant toutes les répartitions distinctes de segments entre lesdits premier et deuxième groupes.

19. Dispositif selon la revendication 17 ou 18, adapté en outre pour construire, pour chaque répartition distincte de segments de séquence d'interrogation, dans la mémoire, une première table d'interrogation indexée par des valeurs possibles des premiers groupes d'interrogation, les entrées dans la première table d'interrogation fournissant un accès à chaque deuxième groupe d'interrogation en utilisant en tant qu'index la valeur d'un premier groupe d'interrogation correspondant.

20. Dispositif selon la revendication 19, adapté en outre pour construire, pour chaque répartition distincte de segments de séquence d'interrogation, dans la mémoire, une deuxième table d'interrogation fournissant un accès à chaque deuxième groupe d'interrogation, les entrées dans la première table d'interrogation fournissant des références vers des entrées appropriées dans la deuxième table d'interrogation.

21. Dispositif selon l'une quelconque des revendications 17 à 20, dans lequel n est au moins égal à deux.

22. Dispositif selon la revendication 21, où n est égal à deux et le constructeur de groupes d'interrogation et le constructeur de groupes cibles sont adaptés chacun pour diviser chaque séquence d'interrogation et fragment de séquence cible en quatre segments.

23. Dispositif selon l'une quelconque des revendications 17 à 22, dans lequel les séquences d'interrogation sont des séquences d'acide nucléique chacune d'une longueur entre environ 16 et 32 bases, et les fragments de séquences cibles sont des fragments de données de génome.

**24.** Dispositif selon la revendication 17, adapté en outre pour effectuer les étapes de procédé selon l'une quelconque des revendications 1 à 14.

102
Start

104
Get New Hash
Functions

No more hash
functions

106
Output Results

Finish

Got hash functions

108
Build Hash
Tables

110
Rewind
Chromosome Files

No more
chromosome
files

112
Get Next
Chromosome File

Got chromosome file

Scan
Chromosome

114

# FIG. 1

## FIG. 2

```
          ┌──────────────┐
          │    Start     │
          └──────┬───────┘
                 │
                 ▼            ┌120
          ┌──────────────┐
          │ Mask Repeats │
          └──────┬───────┘
                 │
                 ▼            ┌122
          ┌──────────────┐
          │ Count Prefixes│
          └──────┬───────┘
                 │
                 ▼            ┌124
          ┌──────────────┐
          │  Hash Query  │
          │  Sequences   │
          └──────┬───────┘
                 │
                 ▼
          ┌──────────────┐
          │    Finish    │
          └──────────────┘
```

## FIG. 3

```
   ┌──────────────┐
   │    Start     │
   └──────┬───────┘
130        │
   ┌───────▼────────┐   No more repeat      ┌134                    No more query        ┌144
   │ Get Next Repeat│   sequences    ┌──────────────────┐          sequences      ┌──────────────────┐
   │   Sequence     │───────────────▶│ Get Next Query   │───────────────────────▶│ Resize MatchStore│
   └───────┬────────┘                │   Sequence       │                         │     To Size      │
           │  Got repeat             └────────┬─────────┘                         └────────┬─────────┘
           │  sequence                        │ Got query                                  │        ┌146
   ┌───────▼────────┐               ┌─────────▼────────┐                          ┌────────▼─────────┐
   │   Add To       │               │  Size=Size+1     │                          │    Delete        │
   │  RepeatList    │               └─────────┬────────┘                          │   RepeatList     │
   └────────────────┘                         │      ┌136                         └────────┬─────────┘
132                  Not a          ┌─────────▼────────┐                                   │
                     repeat         │ Find Query In    │                          ┌────────▼─────────┐
                                    │  RepeatList      │                          │     Finish       │
                                    └─────────┬────────┘                          └──────────────────┘
                                     Is a repeat   ┌138
                                    ┌─────────▼────────┐
                                    │ Resize MatchStore│
                                    │    To Size       │
                                    └─────────┬────────┘
                                              │   ┌140
                                    ┌─────────▼────────┐
                                    │ Flag Entry Size  │
                                    │ Of MatchStore    │
                                    │  As Repeat       │
                                    └──────────────────┘  ┌142
```

FIG. 4

FIG. 5

Start

210 ⌐

Get Next
Chromosome
Fragment

No more
chromosome
fragments

Finish

Got Fragment

Get *Prefix*,
*Suffix*

⌐212

Search For
*Prefix, Suffix*
In *P1, L1*

⌐214

Search For
*Suffix, Prefix*
In *P2, L2*

⌐216

# FIG. 6

Start

220 ⌐

*i=P1 [Prefix]*

222 ⌐

Is *i* equal To
*P1 [Prefix+1]* ?

Yes (no more
suffixes to
check)

Finish

224 ⌐

No

⌐229

⌐230

Compare *L1 [ i ]*
To *Suffix*

Do We Want To
Store This Match?

Yes

Add Match Details
To *MatchStore*

No

*i=i+1*

# FIG. 7

```
                    ┌──────────────────┐
                    │      Start       │
                    └──────────────────┘
                             │
                             ▼
                    ┌──────────────────┐
                    │   i=P2 [Suffix]  │
                    └──────────────────┘
                             │
                             ▼              Yes (no more
              ┌──────────────────────┐      suffixes to
              │      Is i Equal To   │         check)          ┌──────────────────┐
   ┌─────────▶│      P2 [Suffix+1]?  │───────────────────────▶│      Finish      │
   │          └──────────────────────┘                         └──────────────────┘
   │                       │ No
   │                       ▼
   │          ┌──────────────────┐      ┌──────────────────┐  Yes  ┌──────────────────────┐
   │          │  Compare L2 [ i ]│─────▶│  Do We Want To   │──────▶│  Add Match Details   │
   │          │    To Prefix     │      │ Store This Match?│       │    To MatchStore     │
   │          └──────────────────┘      └──────────────────┘       └──────────────────────┘
   │                       │                      │ No                        │
   │                       ▼                      │                           │
   │          ┌──────────────────┐                │                           │
   └──────────│      i=i+1       │◀───────────────┴───────────────────────────┘
              └──────────────────┘
```

# FIG. 8

FIG. 9

EP 1 704 506 B1

FIG. 10

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Non-patent literature cited in the description**

- **WU ; MANBER.** A Fast Algorithm for Multi-Pattern Searching. *Tech Report TR-94-17,* 1994
- **KIM ; KIM.** A fast multiple string-pattern matching algorithm. *Proc. of 17th AoM/IaoM Conf. on Computer Science,* August 1999
- **MUTH ; MANBER.** Approximate Multiple String Search. *Proc. of 7th Annual Symposium on Combinatorial Pattern Matching,* 1996, 75-86